# EUROPEAN PATENT SPECIFICATION

(11) **EP 1 806 411 B1**
(45) Date of publication and mention of the grant of the patent: **17.12.2008**
(21) Application number: 06000158.3
(22) Date of filing: 05.01.2006
(51) Int. Cl.: C12P 23/00

(54) **Process for obtaining zeaxanthin from algae**
Verfahren zur Gewinnung von Zeaxanthin aus Algen
Procédé pour la préparation de la zéaxanthine à partir d'algues

(43) Date of publication of application: 11.07.2007
(73) Proprietor: Cognis IP Management GmbH, 40589 Düsseldorf (DE); Universidad de Sevilla, 41012 Sevilla (ES)
(72) Inventor: Weiss, Dr. Albrecht, 40764 Langenfeld (DE); Johannisbauer, Dr. Wilhelm, 40699 Erkrath (DE); Gutsche, Dr. Bernhard, 40724 Hilden (DE); Martin, Lucia, 41016 Sevilla (ES); F. Cordero, Baldomero, 41930 Bormujos Sevilla (ES); Rodriguez, Prof Dr. Herminia, 41018 Sevilla (ES); Vargas, M. Angeles, 41700 Dos-Hermanes sevilla (ES); Obrastzova, Irina, 41010 Sevilla (ES)
(74) Representative: Fabry, Bernd

(56) References cited:
- LAGARDE D ET AL: "INCREASED PRODUCTION OF ZEAXANTHIN AND OTHER PIGMENTS BY APPLICATION OF GENETIC ENGINEERING TECHNIQUES TO SYNECHOCYSTIS SP. STRAIN PCC 6803" APPLIED AND ENVIRONMENTAL MICROBIOLOGY, WASHINGTON,DC, US, vol. 66, no. 1, 2000, pages 64-72, XP000874121 ISSN: 0099-2240
- GOETZ THOMAS ET AL: "Protection of photosynthesis against ultraviolet-B radiation by carotenoids in transformants of the cyanobacterium Synechococcus PCC7942" PLANT PHYSIOLOGY (ROCKVILLE), vol. 120, no. 2, June 1999 (1999-06), pages 599-604, XP002388740 ISSN: 0032-0889
- DATABASE BIOSIS [Online] BIOSCIENCES INFORMATION SERVICE, PHILADELPHIA, PA, US; July 1999 (1999-07), MASAMOTO KAZUMORI ET AL: "Accumulation of zeaxanthin in cytoplasmic membranes of the cyanobacterium Synechococcus sp. strain PCC7942 grown under high light condition" XP002388780 Database accession no. PREV199900440463 & JOURNAL OF PLANT PHYSIOLOGY, vol. 155, no. 1, July 1999 (1999-07), pages 136-138, ISSN: 0176-1617
- BISWAL BASANTI ET AL: "Changes in carotenoids but not in D1 protein in response to nitrogen depletion and recovery in a cyanobacterium" FEMS MICROBIOLOGY LETTERS, vol. 116, no. 3, 1994, pages 341-347, XP002388741 ISSN: 0378-1097
- JIN EONSEON ET AL: "A mutant of the green alga Dunaliella salina constitutively accumulates zeaxanthin under all growth conditions." BIOTECHNOLOGY AND BIOENGINEERING, vol. 81, no. 1, 5 January 2003 (2003-01-05), pages 115-124, XP002388742 ISSN: 0006-3592
- NOAMAN N H ET AL: "Factors affecting antimicrobial activity of Synechococcus leopoliensis" MICROBIOLOGICAL RESEARCH, FISCHER, JENA,, DE, vol. 159, no. 4, 15 December 2004 (2004-12-15), pages 395-402, XP004956712 ISSN: 0944-5013

## Description

### Object of the invention

The present invention relates to a process for obtaining zeaxanthin from specific algae and the use of specific algae strains for the production of zeaxanthin and zeaxanthin-enriched products.

### State of the art

Zeaxanthin, together with lutein, is an essential component of the macular pigment in the retina of the eye. A low level of intake of this particular carotenoid increases the risk of age-related macular degeneration (AMD) and cataracts, which are the leading causes of visual impairment and acquired blindness, and are key quality of life issues among millions of ageing people. One of the first large-scale studies on carotenoids is the Eye Disease Case Control Study, in which diet was compared to the risk of developing AMD. Results found a significantly lower risk of developing the eye disease in people showing high amounts of lutein and zeaxanthin in their blood. Also, the people who followed a diet with the highest amounts of lutein and zeaxanthin developed a significantly lower risk of AMD than those whose diet contained the least amount (as low as 1.2 mg per day). Dietary studies confirmed the association between frequent consumption of spinach or collard greens, which are particular good sources of lutein and zeaxanthin and of lower AMD risk. Similar results were found in a recent analysis of a US dietary study called the Third National Health and Nutrition Examination Survey or NHANES III. This analysis also showed that consuming lutein and zeaxanthin was associated with a reduced risk of developing AMD. While lutein is relatively abundant in the food we eat, zeaxanthin is not that easily obtained through a well-balanced diet, and thus should be added as a food additive.

Zeaxanthin (3,3'-dihydroxy-β-carotene) represents an oxygenated carotenoid or xanthophyll. The conjugated double bond system determines its colour (yellow-red) and is responsible for the biological action.

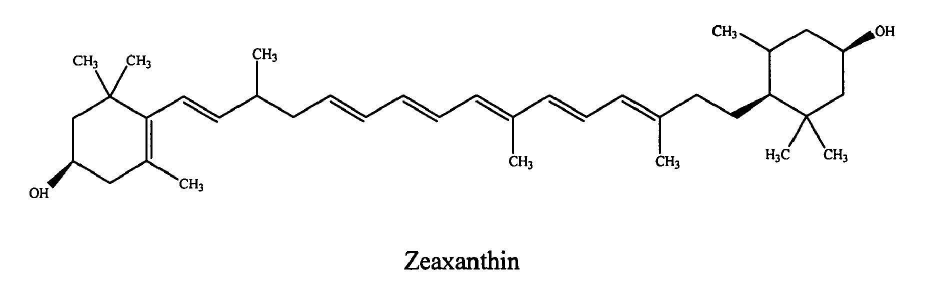

The physiological properties of zeaxanthin and particularly its function as an antioxidant are due to its potential to inactivate singlet oxygen and to quench active radicals. Up to today, zeaxanthin is mainly produced synthetically since the content of this carotenoid in natural sources is considered to be rather low for any industrial production under economic conditions. For example, zeaxanthin is present in some bacteria (e.g. *Flavobacterium, Paracoccus, Halobacterium, Xanthobacter* or *Erwinia*) and higher plants, where they play a role in the so-called Xanthophyll Cycle. In the case of *Flavobacterium* the volumetric content in zeaxanthin is 10.6 mg/l after a six-fold improvement by using different intermediates of the citric acid cycle. With regard to the micro-alga *Neospongiococcum excentricum,* the content of zeaxanthin of the wild type is 0.35 mg/g dry weight. In a constitutive mutant of *Dunaliella salina* the content of zeaxanthin has been found to be about 6 mg/g dry weight [see Jin et al. Biotechnol. Bioeng. 81:115-124 (2002)].

The complex problem underlying the present invention has therefore been to develop a process which allows to obtain zeaxanthin from natural sources in higher yields compared to the state of the art, more particularly from algae showing simultaneously
- a specific growth rate µ at medium light conditions of at least 0.07 h⁻¹ (measured under phototropic conditions using inorganic media);
- a zeaxanthin/lutein ratio of more than 10;
- a factor obtained by multiplication of growth rate and zeaxanthin volumentric productivity of at least 0,01 mgl⁻¹h⁻²;
- a chloropyll a / zeaxanthin ratio of less than 10, and
- a volumetric zeaxanthin productivity of at least 0.04 mg 1⁻¹h⁻¹.

In addition, the starting material should be easy to cultivate and to harvest so that it is possible to carry out the reaction in a photo-bioreactor. Finally, the sources should be free of any harmful toxins or to be cultivated under such non-toxin-producing conditions to avoid harmful toxin formation, and be resistant against contamination.

### Description of the invention

The present invention claims a process for obtaining zeaxanthin, which is characterised in that
(a) blue-green algae selected from the group of strains consisting of *Anacystis nidulans* (L-1402-1), *Synechococcus sp.* (PCC 7942) and *Synechocystis sp.* (PCC 6803) are cultivated in the presence of 0.1 to 20 mM salts of acetic acid to produce zeaxanthin until a desired content of the antioxidant is reached,
(b) the algae are harvested and formulated to a product and/ or
(c) the content of zeaxanthin is separated off from the remaining biomass.

Surprisingly, it had been found that blue-green algae (cyanobacteria) of the cited types, particularly *Anacystis nidulans* (L-1402-1), exhibit a powerful zeaxanthin production fulfilling the complex requirements as outlined above, which allow to obtain the high-value product in a significantly higher amount compared to any other known micro-organism or plant reported in the state of the art. The cited blue-green algae in general, and in particular *Anacystis,* also show a number of additional advantages; compared to other algae they exhibit a quick growth (doubling time from 3.6 to 5.3 h), do not clump and are easily to handle, so that they are suitable for cultivation, especially in tubular photo-bioreactors. They grow auto- or mixotrophically in a very simple and cheap mineral medium and are resistant with respect to contamination.

### Blue-green algae

Among the three strains cited above, *Anacystis nidulans* (L-1402-1) is preferred since it does not only exhibit the highest productivity with respect to zeaxanthin, but also shows the best resistance against pollutants, and the fastest growth rate. Nevertheless, it shall be understood that the present invention is not limited to these wild forms, but also encompasses any mutants of these strains including forms obtained by genetic modification or engineering.

### Cultivation conditions

Many culture conditions and culture media are known for small-scale stock cultures and large-scale cultures of algae cells. However, for the purposes of the present invention, it was found that the optimal culture conditions and media are actually the relatively simple culture conditions and media described herein below, these therefore being the preferred ones in accordance with the invention. For example, temperature is always a critical factor for the growth of algae. It has been found that very favourable conditions are achieved between 20 and 40 °C with an optimum at about 30 °C.

In a further preferred embodiment of the invention, the algae are grown mixotrophically (with additional nutrients to enhance cell growth), more particularly, Arnon medium has been found to support the growth in an optimal manner, preferably if combined with nitrates. The growth rate is increased by the addition of salts of acetic acid, in particular sodium acetate, in amounts of 0.1 to 20, preferably about 15 mM.

Finally, irradiation is also a critical parameter. As far as screening and pilot plant tests are concerned, mercury halide lamps are used and cultivation is carried out under a light irradiation of 500 to 1,500, and preferably of about 1,200 µEm⁻²s⁻¹. For production, of course, natural light conditions are preferred.

In a further preferred embodiment of the present invention, the cells are cultivated in a photo-bioreactor, preferably a tubular or a panel photo-bioreactor, having the advantage of a very large surface area with respect to its volume for optimal large-scale production of algae cells in their growth phase. Usually, such bioreactors have a volume in the range from 100 to 35,000 litres, depending on the scale of production that is desired, and which as integral components contain commercially available tubes made of PVC, acrylic (plexiglass), polycarbonate or glass, having a diameter of about 3 to 5 cm. In operation, the culture cells are circulated through the device in tap water to which CO₂ is added, using a pump. The green cultures from the liquid stock cultures (or inoccula) are inoculated into said photo-bioreactor, in which the algae are sparged with a CO₂ containing gas like a mixture of CO₂ in air or CO₂ as the main gas component. Sparging and pumping also served to prevent clumping of the cells. For production scale in a tubular photo-bioreactor, 90 to 100% pure CO₂ might be applied, also inexpensive CO₂ from industrial plants (e.g. from a quick lime process) are preferred.

The initial cell concentration in the photo-bioreactor during process cultivation is preferably adjusted from about 0.1 to 0.3 * 10⁶ cells/ml at cell concentrations (dry mass) between 0,1 - 8 g dry biomass /l, by dilution with fresh modified cultivation medium. The light intensity is usually kept in the range of between 500 and 1,500 µE*m⁻²s⁻¹ as provided by mercury halide lamps or as natural light. It has been found advantageous to maintain the temperature in the photo-bioreactor in the range between 25 and 28 °C. By using a glass house as an indoor cultivation room it is easy to keep temperatures below 32 °C in central and northern Europe in summer time. In addition, the tubular photo-bioreactor and the indoor placement have the advantage of clean, long term and controlled operation, and if the photo-bioreactor is made of glass tubes instead of plastic it does not require extensive maintenance or revamping in order to operate for a long time. Further, the culture conditions are extremely inexpensive as the cells are grown in a cheap mineral medium of tap water with the addition of carbon dioxide as, essentially, the major nutrient source.

### Harvesting and recovery of zeaxanthin

In order to obtain zeaxanthin or a zeaxanthin-enriched product from the cultivated algae cells, many procedures have been described, for example, the procedures in WO 89/006910. While these procedures may be employed in accordance with the present invention, the preferred procedure is that of centrifugation, or sedimentation or filtration under vacuum to concentrate the cells, and drying of the concentrated cells. The dried cell mass is then preferably stored at low temperatures (e.g., - 20°C or even lower) under oxygen-free conditions, e.g., by vacuum packing or, preferably, by introduction into plastic bags or the like together with nitrogen (N₂) to remove the oxygen.

In another preferred embodiment of the invention, the process comprises the following - additional - steps:
(i) Harvesting the cells cultivated in step (b) by collecting said cells to form a concentrated suspension,
(ii) optionally adding antioxidants and emulsifiers to said suspension, and
(iii) disrupting the collected cells and drying them to obtain a zeaxanthin or a zeaxanthin-enriched product.

Generally, the collection and concentration of said cells is carried out by centrifugation, or sedimentation or filtration under vacuum, and the drying of said cells is done by lyophilisation, combined drying and grinding (air-vortex-mill), or spray drying.

More particularly, while each cultivation cycle optimally lasts about four to six days, the following harvesting procedure is performed based on the fact that algae cells readily sediment once collected from the photo-bioreactor. Thus, the cell biomass from the photo-bioreactor is collected into a standard large volume funnel, e.g. an Imhoff funnel, and left to stand for a few hours (about 3-5 hours) to facilitate sedimentation of the cells. It was found that approximately 30 % b.w. of the total volume of biomass from the bioreactor represented the cell sediment while the remaining approx. 70 % b.w. of the total collected volume represented the tap water used in the cultivation. This tap water can thus be easily collected and used for a new bioreactor inoculation and cultivation procedure (i.e., the originally used tap water is almost completely recyclable). The above precipitated and concentrated cell culture is then collected from the funnel and subjected to centrifugation or vacuum filtration to further concentrate the cells. Routinely, a biomass yield of about 40 % b.w. solids is obtained following the centrifugation step, or about 30 % b.w. solids following vacuum filtration. Here, too, the approximately 60 % b.w. of the total volume subjected to centrifugation, or approximately 70 % b.w. of the total volume subjected to vacuum filtration, being the supernatant volume, could also be collected and used for another round of the cultivation procedure, this supernatant being primarily the original tap water used in the procedure.

The concentrated cell slurry obtained from the above centrifugation step is then homogenized and stabilised by adding anti-oxidants and then dried, preferably by lyophilisation, although spray drying also proved to be effective. Such antioxidants are selected from the group consisting of ethoxyquin, butylated hydroxyanisole, butylated hydroxytoluene (BHT), tocopherols, di-tert-butyl-paracresol and propyl gallate. The preferred antioxidant, however, is a natural tocopherol product containing 30 % b.w. of alpha tocopherol to have at least a real natural product. Usually, the amount of antioxidant added in the grinding procedure will range from about 0.05 to 5 % (w/w) of the amount of dry powder. The powders are packed into a plastic bag pre-filled with nitrogen gas to remove oxygen (which causes pigment oxidation, i.e. degradation of the active) and are then stored at -20 °C prior, e.g., to processing to prepare the food additive.

The final stage of producing a zeaxanthin or a zeaxanthin-enriched product in the form of small particles easily digested by humans or animals may also be carried out in a number of ways as previously described in the art. Thus, zeaxanthin and other algae components are processed to assure a high bioavailability. The preferred procedure involves the use of a standard ball mill in which the biomass slurry is disintegrated as a suspension in water in the presence of any suitable anti-oxidant to prevent oxidation of the zeaxanthin. After drying, this yields a powder-like product of small particle size.

The powder thus obtained may then be utilized directly or in admixture with other ingredients as an additive to fish meal for the sake of coloration or in food applications like dietary supplements. In another process, the zeaxanthins can be concentrated by an extraction process including extraction with supercritical solvents to use them for formulation of food supplement or pharmaceutical products.

### Industrial application

According to the teaching of the present invention, certain blue-green algae have been found to exhibit a surprisingly high productivity for the production of zeaxanthin, especially if cultivated under optimised conditions. Another object of the present disclosure is therefore directed to the use of blue green algae selected from the group of strains consisting of *Anacystis nidulans* (L-1402-1), *Synechococcus sp.* (PCC 7492) and *Synechocystis sp.* (PCC 6803), either in their wild forms or in the form of any mutant, including those forms obtained from by genetic modification or engineering for the production of zeaxanthin or zeaxanthin-enriched products. Finally, the present disclosure covers the use of zeaxanthin or zeaxanthin-enriched products, including the zeaxanthin enriched biomass (as directly obtained from the cultivation) as a food or feed additive or a cosmetic or pharmaceutical raw material.

### Examples

### Zeaxanthin analysis

For the analysis of zeaxanthin, the pigments were extracted with methanol at 70 °C, centrifuged, the supernatant evaporated under N₂ at 30 °C and the pellet re-suspended in acetone, centrifuged and analyzed by HPLC using a Waters Spherisorb S5 ODS1 4.6 x 250 mm cartridge column. The pigments were detected by using a photodiode-array detector.

### Cell growth conditions

Stock cultures of micro-algae were grown photoautotrophically in batch culture, in 100 ml of either Arnon culture medium (Arnon medium, modified to contain 4 mM K₂HPO₄ and 20 mM NaNO₃) or BG11c medium (Blue-green medium, www.ccap.ac.uk) in conical flasks of 200 ml capacity and illuminated with fluorescent lamps at 92 µE m⁻²s⁻¹. Culture temperature was 30 °C. The screening of the strains for the production of zeaxanthin was performed under the following conditions:
- Photoautotrophic batch cultures in Roux Flasks (750 ml) were started with cells at the exponential phase from the stock cultures at 0.7-0.8 mg (chlorophyll) l⁻¹
- Irradiance: Continuous, 920-1610 µEm⁻²s⁻¹ (mercury halide lamps)
- Temperature: 30 °C Bubbling: The cultures were bubbled with air supplemented with 1 %(v/v) CO₂.
- Culture medium: The same as in the case of stock culture.

Several parameters were analyzed in the different micro-algae, as cell density, dry weight, carotenoids and chlorophylls, pH, cell morphology and the specific growth rate (µ). Growth was followed by determining the chlorophyll A content (mgl⁻¹), or by dry weight (gl⁻¹). For dry weight determination, 5 ml aliquots were filtered through previously weighted 0.45 µm diameter Millipore filters, washed twice with distilled water and dried at 80 °C for 24 h. Chlorophyll *a* was determined spectrophotometrically in methanol extracts employing the extinction coefficient given by Mackinney. For mixotrophic growth, cultures were supplemented with 15 to 20 mM sodium acetate (experimental) and for nitrate optimization the media were supplemented with 10 to 40 mM sodium nitrate. For the optimization of irradiance, light irradiances from 92 µm⁻²s⁻¹ to 1610 µE m⁻²s⁻¹ were assayed. Temperature ranged from 22 °C to 40 °C in the experiment for temperature optimization. The medium was optimised for *Anacystis nidulans* L-1402; the following media were used: BG11c, Arnon medium supplemented with 20 mM nitrate, and BP medium (Jaworski's medium, catalogue of strains CCAP).

### Selection of blue-green micro-algae strains

The following Tables 1 and 2 show a comparison of different blue-green micro-algae strains with respect to the productivity for zeaxanthin (P) and the factor "specific growth rate" multiplied with "zeaxanthin productivity". As outlined above, the algae were cultivated in BG11c Medium at 30 °C, the irradiance was 350 Wm⁻² (1610 µEm⁻²s⁻¹). Only three of them show a high content in zeaxanthin, both at the exponential phase of growth and at the end of the culture: *Anacystis nidulans* L-1402-1, *Synechococcus sp.* PCC 7942 and *Synechocystis sp.* PCC 6803. The three strains also exhibited the highest specific growth rates. Therefore, these strains present the highest zeaxanthin productivities (from 3.0 to 7.9 mg 1⁻¹ day⁻¹).

**Table 1**

| **Productivity of blue-green micro-algae for zeaxanthin** | | | | | |
|---|---|---|---|---|---|
| **Strains /Results** | **µ** [h⁻¹] | **Chl A** [mgl⁻¹] | **DW (max)** [gl⁻¹] | **Zea (max)** [mgl⁻¹] | **P (Zea)** [mgl⁻¹h⁻¹] |
| *Anabaena sp. PCC 7120* | 0.05 | 13.26 | 1.06 | 0.06 | 0.00 |
| *Anacystis nidulans L-1402-1* | 0.16 | 23.30 | | 11.20 | 0.33 |
| *Chlorogloeopsis sp. PCC 6912* | 0.02 | 12.80 | 3.90 | 1.10 | 0.01 |
| *Dermocarpa sp. PCC 743 7* | 0.03 | 14.00 | 2.80 | 1.60 | 0.01 |
| *Nostoc caquena* | 0.02 | 2.90 | 6.80 | 0.20 | |
| *Nodularia chucula* | 0.05 | 32.20 | 2.50 | 0.70 | 0.02 |
| *Nostoc llaita* | 0.02 | 56.60 | 11.20 | 0.70 | 0.00 |
| *Nostoc sp. PCC 9201* | 0.03 | 23.30 | 2.80 | 0.30 | 0.00 |
| *Nostoc paludosum PCC 9206* | 0.06 | 38.10 | 4.40 | 0.01 | |
| *Synechococcus sp. PCC 7492* | 0.08 | 9.60 | | 4.70 | 0.12 |
| *Synechocystis sp. PCC 6803* | 0.08 | 22.40 | 1.50 | 3.00 | 0.20 |

| | | | | | |
|---|---|---|---|---|---|
| Chl A = chlorophyll A; Zea = zeaxanthin; Lut = lutein; DW = dry weight, P = productivity | | | | | |

**Table 2**

| **Specific Growth Rate * Productivity of blue-green micro-algae for zeaxanthin** | | | | |
|---|---|---|---|---|
| **Strains / Results** | **Chl A** **Zea (max)** | **Zea (max)** **Lut (max)** | **Zea (max)** **DW (max)** | **SPGR *** **P(Zea)** |
| *Anabaena sp. PCC 7120* | 221.00 | 1.20 | 0.06 | 0.000 |
| *Anacystis nidulans L-1402-1* | 2.08 | 14.00 | | 0.053 |
| *Chlorogloeopsis sp. PCC 6912* | 11.64 | 1.22 | 0.28 | 0.000 |
| *Dermocarpa sp. PCC 743 7* | 8.75 | 2.00 | 0.57 | 0.000 |
| *Nostoc caquena* | 14.50 | | 0.03 | 0.000 |
| *Nodularia chucula* | 46.00 | 3.50 | 0.28 | 0.001 |
| *Nostoc llaita* | 83.71 | 0.47 | 0.06 | 0.000 |
| *Nostoc sp. PCC 9201* | 77.67 | 0.14 | 0.11 | 0.000 |
| *Nostoc paludosum PCC 9206* | 3810.00 | | 0.000 | 0.000 |
| *Synechococcus sp. PCC 7492* | 2.04 | 15.16 | | 0.010 |
| *Synechocystis sp. PCC 6803* | 7.47 | 2.50 | 2.00 | 0.016 |

| | | | | |
|---|---|---|---|---|
| Chl A = chlorophyll A; Zea = zeaxanthin; Lut = lutein; DW = dry weight, SGR = Specific Growth Rate | | | | |

### Kinetics of the accumulation of carotenoids

Table 3 shows the kinetics of accumulation of different carotenoids in *Anacystis nidulans* L-1402-1, which was selected for further testing due to its particular high productivity of zeaxanthin. Zeaxanthin is the major carotenoid, but other carotenoids are present either in very low amounts or not detected at all. Zeaxanthin in the cultures increased with time, changing from 1.9 at the exponential phase to 11.2 mg l⁻¹ in the stationary phase. Total carotenoids followed the same trend, increasing from 2.5 to 14.7 mg l⁻¹ from the exponential to the stationary phase of culture.

### Effect of growth medium

In the following Tables 4, 5 and 6, optimized parameters with respect to culture medium, irradiance and acetate concentration for *Anacystis nidulans* are given. The highest cell growth, measured as chlorophyll content in the cultures, was obtained by using Arnon medium supplemented with 20 mM of nitrate, whereas the lowest growth was observed with BG11c medium. In particular, it was observed that the specific growth rate was higher in BG11c than in Arnon and BP medium, however, the highest zeaxanthin content was found in Arnon medium. Therefore, the highest zeaxanthin production occurred when cells were grown with Arnon medium, supplemented with 20 mM nitrate.

**Table 3**

| **Kinetics of the accumulation of carotenoids [mgl-1]** | | | | | | |
|---|---|---|---|---|---|---|
| **Time [h]** | **Exponential phase** | | **Deceleration phase** | | **Stationary phase** | |
| **Carotenoids** | 12.5 | 18.5 | 24.5 | 38.0 | 94.5 | 170 |
| Violaxanthin | - | - | - | - | - | - |
| Astaxanthin | - | - | - | - | - | - |
| Anteraxanthin | 0.04 | 0.04 | 0.08 | 0.09 | 0.07 | 0.07 |
| Lutein | 0.06 | 0.07 | 0.10 | 0.14 | 0.61 | 0.82 |
| Zeaxanthin | 1.90 | 2.80 | 3.50 | 4.80 | 8.50 | 11.20 |
| Canthaxanthin | - | - | - | - | - | - |
| β-Cryptoxanthin | 0.14 | 0.07 | 0.11 | 0.16 | 0.23 | 0.30 |
| Lycopene | - | - | - | - | - | - |
| α-Carotene | 0.26 | 0.54 | 0.90 | 1.30 | 1.80 | 1.60 |
| β-Carotene | 0.07 | 0.12 | 0.22 | 0.43 | 0.80 | 0.74 |
| **Total Carotenoids** | **2.5** | **3,6** | **4,9** | **6.9** | **12.0** | **14.7** |

**Table 4**

| **Effect of growth medium** | | | | | | |
|---|---|---|---|---|---|---|
| **Medium** | **µ** [h⁻¹] | **Zea (max-E)*** [mgl⁻¹] | **Chl (max)** [mgl⁻¹] | **Zea (max)** [mgl⁻¹] | **Zea Production** | |
| | | | | | mgl⁻¹h⁻¹ | mgl⁻¹d⁻¹ |
| BG11c | 0.19 | 2.3 | 12.0 | 6.5 | 0.44 | 10.6 |
| Arnon | 0.13 | 4.8 | 67.9 | 16.2 | 0.62 | 15.0 |
| BP | 0.12 | 4.8 | 25.9 | 8.7 | 0.58 | 13.9 |

| | | | | | | |
|---|---|---|---|---|---|---|
| *) Maximal zeaxanthin content in exponential phase | | | | | | |

In addition, it was observed that in the kinetics of zeaxanthin accumulation by *Anacystis nidulans* until the end of exponential phase, the accumulation of zeaxanthin in BP and Arnon medium was similar and higher than in BG11c medium. Afterwards, the culture performed with Arnon medium accumulated much more zeaxanthin than the other cultures.

### Effect of light irradiance

Table 5 reflects the effect of light irradiance on the zeaxanthin production for *Anacystis nidulans.* The optimum light irradiance for zeaxanthin production has found to be 1,150 µE m⁻²s⁻¹. The highest specific growth rates, zeaxanthin contents in mg l⁻¹ and zeaxanthin productions were found at irradiances from 1,610 to 690 µE m⁻² s⁻¹, productivity decreasing to half or less when irradiance was lowered from 690 to 460 µE m⁻²s⁻¹. The same trend was observed with regard to chlorophyll and zeaxanthin content in mg g⁻¹ dry weight.

**Table 5**

| **Effect of light irradiance** | | | | | | |
|---|---|---|---|---|---|---|
| **Irradiance** [µEm⁻²s⁻¹] | **µ** [h⁻¹] | **Chl (max)** [mgl⁻¹] | **Zea (max)** [mgl⁻¹] | **Zea (max)** [mgg⁻¹DW] | **Zea (Production)** | |
| | | | | | mgl⁻¹h⁻¹ | mgl⁻¹d⁻¹ |
| 1,610 | 0.14 | 45.7 | 13.3 | 2.9 | 0.42 | 10.1 |
| 1,150 | 0.14 | 48.7 | 13.3 | 2.8 | 0.53 | 12.7 |
| 690 | 0.13 | 56.1 | 13.2 | 2.9 | 0.47 | 11.3 |
| 460 | 0.10 | 41.2 | 7.0 | 2.2 | 0.21 | 5.0 |
| 184 | 0.06 | 31.0 | 4.4 | 1.9 | 0.13 | 3.1 |
| 92 | 0.07 | 22.3 | 2.9 | 1.5 | 0.09 | 2.2 |

### Effect of sodium acetate

Table 6 shows the effect of sodium acetate on growth, zeaxanthin content and zeaxanthin productivity in *Anacystis nidulans.* Specific growth rate was kept at about 0.1 h⁻¹ from 0 to 20mM acetate, cells dieing at 30 mM acetate. Maximum zeaxanthin in the exponential phase and at the end of the culture were kept around the same value from 0 to 15 mM acetate, decreasing at higher acetate concentrations. Optimum acetate in the medium was 15 mM, since zeaxanthin productivity was enhanced by a 27 % when compared with photoautotrophic cultures.

**Table 6**

| **Effect of sodium acetate** | | | | | | |
|---|---|---|---|---|---|---|
| **Na-acetate** [mM] | **µ** [h⁻¹] | **Chl (max)** [mgl⁻¹] | **Zea (max)** [mgl⁻¹] | **Zea (max)** [mgg⁻¹DW] | **Zea (Production)** | |
| | | | | | mgl⁻¹h⁻¹ | mgl⁻¹d⁻¹ |
| 0 | 0.11 | 45.5 | 11.7 | 2.9 | 0.53 | 12.7 |
| 15 | 0.12 | 37.2 | 11.5 | 3.4 | 0.67 | 16.1 |
| 20 | 0.09 | 69.8 | 9.05 | 3.3 | 0.01 | 0.34 |

## Claims

1. A process for obtaining zeaxanthin from algae, **characterised in that**
(a) blue-green algae selected from the group of strains consisting of *Anacystis nidulans* (L-1402-1), *Synechococcus sp.* (PCC 7942) and *Synechocystis sp.* (PCC 6803) are cultivated in the presence of 0.1 to 20 mM salts of acetic acid - calculated on the cultivation media - to produce zeaxanthin until a desired content of the antioxidant is reached,
(b) the algae are harvested and used as a product and/or
(c) the content of zeaxanthin is separated off from the remaining biomass.

2. Process according to claim 1, **characterised in that** said blue-green algae represent wild forms or any mutants of these strains including those forms obtained by genetic modification or engineering.

3. Process according to claims 1 and/or 2, **characterised in that** cultivation of the algae is conducted at a temperature from 20 to 40 °C.

4. Process according to any of the claims 1 to 3, **characterised in that** cultivation is conducted using a mixotrophic culture medium.

5. Process according to any of claims 1 to 4, **characterised in that** cultivation is conducted under a light irradiation from 500 to 1,500 µEm⁻²s⁻¹.

6. Process according to any of claims 1 to 5, **characterised in that** cultivation is conducted in a photo-bioreactor.

7. Process according to any of claims 1 to 6, **characterised in that** the cells cultivated in step (b) are collected to form a concentrated suspension, optionally antioxidants and emulsifiers are added to said suspension, and the collected cells are disrupted and dried to obtain a zeaxanthin or a zeaxanthin-enriched product.

## Patentansprüche

1. Verfahren zur Gewinnung Zeaxanthin aus Algen, **dadurch gekennzeichnet, daß** man
(a) Blaualgen aus der Gruppe der Stämme *Anacystis nidulans* (L-1402-1), *Synechococcus sp*. (PCC 7942) und *Synechocystis sp*. (PCC 6803) in Gegenwart von 0,1 bis 20 mM Salzen der Essigsäure - in bezug auf die Kulturmedien - kultiviert, um Zeaxanthin zu produzieren, bis ein gewünschter Gehalt des Antioxidans erreicht ist,
(b) die Algen erntet und als Produkt verwendet und/oder
(c) den Zeaxanthingehalt von der verbleibenden Biomasse abtrennt.

2. Verfahren nach Anspruch 1, **dadurch gekennzeichnet, daß** die Blaualgen Wildformen oder beliebige Mutanten dieser Stämme einschließlich der durch genetische Modifizierung oder Gentechnik erhaltene Stämme darstellen.

3. Verfahren nach Anspruch 1 und/oder 2, **dadurch gekennzeichnet, daß** die Kultivierung der Algen bei einer Temperatur von 20 bis 40°C erfolgt.

4. Verfahren nach Anspruch 1 und/oder 3, **dadurch gekennzeichnet, daß** die Kultivierung mit einem mixotrophen Kulturmedium durchgeführt wird.

5. Verfahren nach Anspruch 1 und/oder 4, **dadurch gekennzeichnet, daß** die Kultivierung unter einer Lichtbestrahlung von 500 bis 1500 µEm⁻²s⁻¹ durchgeführt wird.

6. Verfahren nach einem der Ansprüche 1 bis 5, **dadurch gekennzeichnet, daß** die Kultivierung in einem Photobioreaktor durchgeführt wird.

7. Verfahren nach einem der Ansprüche 1 bis 6, **dadurch gekennzeichnet, daß** man die in Schritt (b) kultivierten Zellen gewinnt, um zu einer konzentrierten Suspension zu gelangen, die Suspension gegebenenfalls mit Antioxidantien und Emulgatoren versetzt und die gewonnenen Zellen aufgeschlossen und getrocknet werden, um zu Zeaxanthin oder einem mit Zeaxanthin angereicherten Produkt zu gelangen.

## Revendications

1. Procédé d'obtention de zéaxanthine à partir d'algues, **caractérisé en ce que :**
(a) des algues bleu-vert choisies dans le groupe de souches constitué d'*Anacystis nidulans* (L-1402-1), de *Synechococcus sp*. (PCC 7942) et de *Synechocystis sp*. (PCC 6803) sont mises en culture en présence de 0,1 à 20 mM de sels d'acide acétique, calculés par rapport aux milieux de culture, pour produire de la zéaxanthine jusqu'à ce qu'une teneur en antioxydant souhaitée soit atteinte,
(b) les algues sont recueillies et utilisées comme produit et/ou
(c) la partie zéaxanthine est séparée de la biomasse restante.

2. Procédé selon la revendication 1, **caractérisé en ce que** lesdites algues bleu-vert représentent les formes sauvages ou tout mutant de ces souches, y compris les formes qui sont obtenues par modification génétique ou génie génétique.

3. Procédé selon les revendications 1 et/ou 2, **caractérisé en ce que** la mise en culture des algues est réalisée à une température comprise entre 20 et 40 °C.

4. Procédé selon l'une quelconque des revendications 1 à 3, **caractérisé en ce que** la mise en culture est réalisée en utilisant un milieu de culture mixotrophe.

5. Procédé selon l'une quelconque des revendications 1 à 4, **caractérisé en ce que** la mise en culture est réalisée sous un rayonnement lumineux compris entre 500 et 1 500 µEm⁻²s⁻¹.

6. Procédé selon l'une quelconque des revendications 1 à 5, **caractérisé en ce que** la mise en culture est réalisée dans un photobioréacteur.

7. Procédé selon l'une quelconque des revendications 1 à 6, **caractérisé en ce que** les cellules mises en culture dans l'étape (b) sont recueillies de manière à former une suspension concentrée, **en ce que** des antioxydants et des agents émulsionnants sont éventuellement ajoutés à ladite suspension et **en ce que** les cellules recueillies sont rompues et séchées de manière à obtenir une zéaxanthine ou un produit enrichi en zéaxanthine.
